(19) Europäisches Patentamt
European Patent Office
Office européen des brevets

(11) Publication number: **0 496 134 A1**

# (12) EUROPEAN PATENT APPLICATION

(21) Application number: **91203327.1**

(51) Int. Cl.$^5$: **A61K 31/565**, A61K 31/58

(22) Date of filing: **17.12.91**

(30) Priority: **21.12.90 EP 90314178**

(43) Date of publication of application:
**29.07.92 Bulletin 92/31**

(84) Designated Contracting States:
**AT BE CH DE DK ES FR GB GR IT LI LU MC NL SE**

(71) Applicant: **AKZO N.V.**
**Velperweg 76**
**NL-6824 BM Arnhem(NL)**

(72) Inventor: **Kellock, John**
**27 Sycamore Drive**
**Hamiltonml 3 7HF, Scotland(GB)**
Inventor: **Taylor, Robert**
**14 McKenna Drive**
**Airdrie ML6 0JF, Scotland(GB)**
Inventor: **Campbell, John**
**3 Allanton Holdings**
**Allanton Shotts ML7 5DO, Scotland(GB)**
Inventor: **Winslow, Eileen**
**21 Loch Laxford**
**East Kilbridge G74 2DL, Scotland(GB)**

(74) Representative: **Hermans, Franciscus G.M. et al**
**P.O. Box 20**
**NL-5340 BH Oss(NL)**

## (54) Use of 2 beta, 16 beta-diamino-3 alpha, 17-dihydroxy androstane derivatives for the treatment of arrhythmic disorders.

(57) The invention concerns the use of a 2$\beta$,16$\beta$-diamino-3$\alpha$,17-dihydroxy androstane derivative of the formula

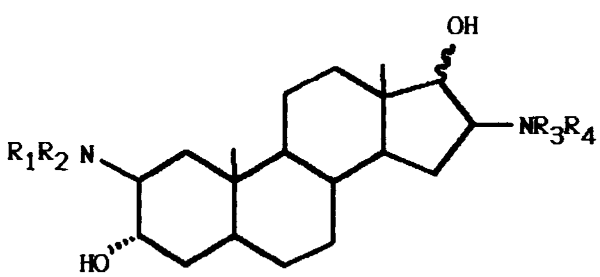

I

in which $R_1$, $R_2$, $R_3$, and $R_4$ are independently selected from hydrogen, ($C_{1-6}$) alkyl, ($C_{7-12}$) aralkyl, ($C_{2-6}$) acyl, and ($C_{3-7}$) cycloalkyl which is optionally substituted with ($C_{1-6}$) alkyl, or $R_1$ and $R_2$, and/or $R_3$ and $R_4$ together with the nitrogen atom to which they are bonded represent a 5-, 6-, or 7-membered ring which is optionally substituted with ($C_{1-6}$) alkyl, and the twitched line represents an $\alpha$ or $\beta$ bond, or pharmaceutically acceptable salts thereof, for the manufacture of a medicament for treating arrhythmic disorders.

EP 0 496 134 A1

The invention concerns the use of 2β,16β-diamino-3α,17-dihydroxy androstane derivatives for the preparation of a medicament for the treatment of arrhythmic disorders. The invention is further related to 2β,16β-diamino-3α,17-dihydroxy androstane derivatives for use in therapy and to pharmaceutical compositions containing the same.

Several 2β,16β-diamino-3α,17-dihydroxy androstane derivatives are known. In USP 3,553,212, EP 287,150, and EP 330,253, for instance, 2β,16β-diamino-3α,17-dihydroxy androstane derivatives having neuromuscular blocking activity are disclosed. Other 2β,16β-diamino-3α,17-dihydroxy androstane derivatives are disclosed as intermediates for the preparation of neuromuscular blocking agents, for example in DE 2,337,882, BE 344,643, EP 288,102, and in Buckett et al., J. Med Chem., 16, 1116 (1973). The synthesis of 3α-amino-2β,17β-dihydroxy-16β-piperidino-5α-androstane was disclosed by Xu et al. in Youji Huaxue (1989), 9, 451 (Chem. Abstr. (1990), 113, 78806p), however, without disclosing a therapeutic effect or a pharmaceutical use thereof.

It has now been found that 2β,16β-diamino-3α,17-dihydroxy androstane derivatives of the formula

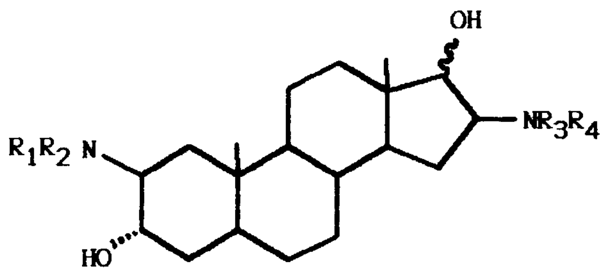

I

in which $R_1$, $R_2$, $R_3$, and $R_4$ are independently selected from hydrogen, $(C_{1-6})$ alkyl, $(C_{7-12})$ aralkyl, $(C_{2-6})$ acyl, and $(C_{3-7})$ cycloalkyl which is optionally substituted with $(C_{1-6})$ alkyl, or $R_1$ and $R_2$, and/or $R_3$ and $R_4$ together with the nitrogen atom to which they are bonded represent a 5-, 6-, or 7-membered ring which is optionally substituted with $(C_{1-6})$ alkyl, and the twitched line represents an α or β bond, or pharmaceutically acceptable salts thereof, can be used for the preparation of a medicament for the treatment of arrhythmic disorders.

The term $(C_{1-6})$ alkyl, as used in the definition of formula I, means a branched or unbranched alkyl group with 1-6 carbon atoms, such as methyl, ethyl, propyl, isopropyl, butyl, *tert*-butyl, pentyl, isopentyl, hexyl and the like. Preferred are alkyl groups with 1-4 carbon atoms, like methyl and *tert*-butyl.

The term $(C_{7-12})$ aralkyl means an aralkyl group with 7-12 carbon atoms, the alkyl moiety of which is derived from a $(C_{1-6})$ alkyl group as defined previously, and the aryl group is a phenyl or naphthyl group, which may be optionally substituted with hydroxy, alkyl (having 1-4 carbon atoms), alkoxy (having 1-4 carbon atoms), or halogen (F, Cl, or Br). The preferred aralkyl group is phenylmethyl.

The term $(C_{2-6})$ acyl means an acyl group which is derived from an aliphatic carboxylic acid with 2-6 carbon atoms, like acetic acid, propionic acid, butyric acid, and the like. The acetyl is the most preferred acyl group.

The term $(C_{3-7})$ cycloalkyl means a cycloalkyl group with 3-7 carbon atoms, such as cyclopropyl, cyclobutyl, cyclopentyl, cyclohexyl, and cycloheptyl. The cycloalkyl group may be substituted with a $(C_{1-6})$ alkyl group. The 5-, 6-, and 7-membered rings which $R_1$ and $R_2$, and $R_3$ and $R_4$ may form together with the nitrogen atom to which they are bonded, may contain a second hetero atom and may be substituted with a $(C_{1-6})$ alkyl group as defined previously. Preferred 5-, 6-, and 7-membered rings are the pyrrolidinyl, piperidinyl, morpholinyl, N-methylpiperazinyl, and perhydroazepinyl rings.

A major cause of death is sudden cardiac arrest, which means that a strong clinical need exists for effective medicaments for the treatment of arrhythmic disorders. To date, the only drugs which have been adequately studied in large multicentre trials, using sudden death as the endpoint, are β-blocking drugs. The most notable examples of β-blockers are propanolol, timolol, and d-sotalol. Sodium channel blocking agents, such as flencainide, encainide and moricizine have also been shown to reduce mortality. Amiodarone, a drug generally thought by clinicians to be the most efficacious antiarrhythmic agent available up to now, is limited in its clinical use because of a number of often unacceptable side-effects. Many of the side-effects appear to be unrelated to its antiarrhythmic action, for example, amiodarone is insoluble and precipitates in the cornea causing visual disturbances, and it inhibits phospholipases resulting in lipidosis which may contribute to other unwanted effects such as pneumonitis, CNS disturbances and photosensitivity.

The invention thus provides compounds which are suitable for the preparation of antiarrhythmic

pharmaceutical compositions, that are devoid of unwanted side-effects associated with known antiarrhythmic agents.

Preferred compounds for the preparation of anti-arrhythmic pharmaceutical compositions are 2$\beta$,16$\beta$-diamino-3$\alpha$,17-dihydroxy androstane derivative of formula I in which $R_1$ and $R_2$ are independently selected from hydrogen, ($C_{1-6}$) alkyl, ($C_{6-12}$) aralkyl, ($C_{2-6}$) acyl, and ($C_{3-7}$) cycloalkyl, or $R_1$ and $R_2$ together with the nitrogen atom to which they are bonded represent a pyrrolidinyl, piperidinyl, perhydroazepinyl, or N-methylpiperazinyl, and $R_3$ and $R_4$ are independently selected from hydrogen and ($C_{1-6}$) alkyl, or $R_3$ and $R_4$ together with the nitrogen atom to which they are bonded represent a pyrrolidinyl, piperidinyl, or perhydroazepinyl, and the twitched line represents an $\alpha$ or $\beta$ bond, or pharmaceutically acceptable salts thereof.

More preferred are compounds and pharmaceutical compositions, the active constituent of which is such a compound, which are not known to have other pharmacological effects. Such compounds are 2$\beta$,16$\beta$-diamino-3$\alpha$,17-dihydroxy androstane derivatives having formula I, in which $R_1$ and $R_2$ are independently selected from hydrogen, ($C_{1-6}$) alkyl, ($C_{7-12}$) aralkyl, ($C_{2-6}$) acyl, and ($C_{3-7}$) cycloalkyl which is optionally substituted with ($C_{1-6}$) alkyl, and $R_3$ and $R_4$ together with the nitrogen atom to which they are bonded represent a 5-, 6-, or 7-membered ring which is optionally substituted with ($C_{1-6}$) alkyl, and the twitched line represents an $\alpha$ or $\beta$ bond, or pharmaceutically acceptable salts thereof.

The most preferred pharmaceutical compositions contain pharmaceutically acceptable auxiliaries and a 2$\beta$,16$\beta$-diamino-3$\alpha$,17-dihydroxy androstane derivative of the formula I, in which $R_1$ is hydrogen, $R_2$ is hydrogen or *tert*-butyl, $R_3$ and $R_4$ together with the nitrogen atom to which they are bonded represent piperidinyl, and the twitched line is a $\beta$ bond.

The compounds of the invention which are known may be prepared according to the methods described in the references cited previously, which are included by reference, or by analogous methods known in the art.

The compounds of formula I may be isolated from the reaction mixture in the form of a pharmaceutically acceptable salt. The pharmaceutically acceptable salts may also be obtained by treating the free base of formula I with an organic or inorganic acid such as HCl, HBr, HI, $H_2SO_4$, $H_3PO_4$, acetic acid, propionic acid, glycolic acid, maleic acid, malonic acid, methanesulphonic acid, fumaric acid, succinic acid, tartaric acid, citric acid, benzoic acid, and ascorbic acid.

As an example, (2$\beta$,3$\alpha$,5$\alpha$,16$\beta$,17$\beta$)-2-amino-16-(1-piperidinyl)-androstane-3,17-diol hydrochloride (1:2) can be prepared by refluxing of 2$\alpha$,3$\alpha$,16$\alpha$,17$\alpha$-diepoxy-17$\beta$-acetoxy-5$\alpha$-androstane in glycol with aqueous piperidine, followed by the reduction of the formed 17-keto group with sodium borohydride, after which the 2,3-epoxy ring is opened by benzylamine and the benzyl group is cleaved by hydrogenation, after which the (2$\beta$,3$\alpha$,5$\alpha$,16$\beta$,17$\beta$)-2-amino-16-(1-piperidinyl)-androstane-3,17-diol obtained is converted into its hydrochloric salt by treatment with hydrochloric acid. The other compounds of the invention can be prepared in an analogous manner.

The compounds of the invention may be administered enterally or parenterally in a daily dosage of 0,001-100 mg per kg body weight, and for humans preferably in a daily dosage of 0,05-20 mg per kg body weight. Mixed with pharmaceutically suitable auxiliaries, e.g. as described in the standard reference, Chase et al., Remington's Pharmaceutical Sciences, the compounds may be compressed into solid dosage units, such as pills, tablets, or be processed into capsules or suppositories.

By means of pharmaceutically suitable liquids the compounds can also be applied as an injection preparation in the form of a solution, suspension, emulsion, or as a spray, e.g. a nasal spray.

The compounds of the invention can be used prophylactically or therapeutically for the treatment of patients suffering from arrhythmic disorders, especially prophylactically to prevent sudden death. The oral and intravenous routes of administration are then preferred.

The invention is further illustrated by the following examples.

Example 1

*In vivo* test

Monophasic ventricular electrocardiograms (MAP) were recorded from an electrode positioned on the epicardium midway between the apex and the base of the left ventricle in the anaesthetized guinea-pig. Bipolar electrodes, hooked into the left ventricle just under the atrium, were used to pace the ventricles at a rate (4.7-6.3 Hz) 5% above normal sinus rhythm. Test drugs were given via a jugular vein at 16 minute intervals and measurements made 15-16 minutes after administration of each dose. The $ED_{50}$ value for MAP prolongation during pacing is defined as the dose of the test compound which prolongs MAP by an

amount equal to half the diastolic interval. (For data see Table).

Example 2

*In vitro* test

Transmembrane cellular action potentials were recorded from isolated guinea-pig papillary muscle driven at 1Hz. The parameters measured were the time taken to reach 90% repolarisation ($APD_{90}$) and the maximum rate of depolarisation ($V_{max}$), obtained by electronic differentation. Action potentials were recorded prior to and 20-30 minutes after addition of test compound to the superfusate. The cumulative method of drug addition was used (1-100 µM) and a single impalement maintained throughout each experiment. EC values for prolongation of $APD_{90}$ (class III action) and reduction of $V_{max}$ (class I action) were calculated from dose/response curves. EC ratios (R) were assessed by calculating the quotient $EC_{25}(V_{max})/EC_{25}(ADP_{90})$ (For data see Table). The higher the value, the higher the class III activity.

**Table**

| I | | | MAP | $APD_{90}$ | | |
|---|---|---|---|---|---|---|
| $NR_1R_2$ | $NR_3R_4$ | 17OH | $ED_{50}$ (mg/kg) (*in vivo*) | $EC_{25}$ (*in vitro*) | $EC_{100}$ (µM) | R |
| 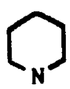 | 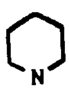 | β | 3,8 | 6,0 | 34 | 3,4 |
| $NHCH_3$ | 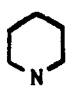 | β | 4,4 | 6,2 | 50 | 3,9 |
| $NH_2$ | 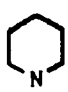 | β | 4,7 | 7,0 | 42 | 2,1 |

| | | | | | | |
|---|---|---|---|---|---|---|
| $N(CH_3)_2$ | 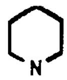 | β | 6,7 | 18,7 | * | 1,5 |
| $NHC(CH_3)_3$ | 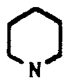 | β | 3,2 | 14,8 | 75 | 1,9 |
| 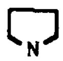 | 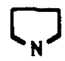 | β | 10,0 | not tested | | |
| 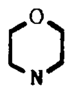 | 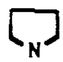 | β | 15,3 | 31,0 | * | 0,6 |
| 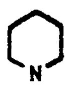 | 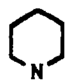 | α | 4,7 | 34,2 | * | 1,1 |
| 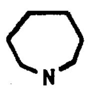 | 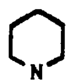 | β | 1,9 | 3,3 | ~100 | 5,6 |
| 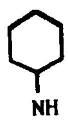 | 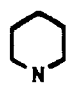 | β | 2,2 | 8,1 | 56 | 0,1 |
| 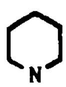 | $NHCH_3$ | β | 2,1 | 26,6 | * | ** |
| $NHCH_3$ | 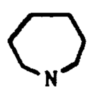 | β | 5,0 | 13,5 | 92 | 3,1 |
| 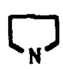 | $NHCH_3$ | β | 3,9 | 1,5 | 54 | ** |
| $N(COCH_3)CH_3$ | 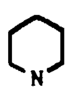 | β | ~10 | 14,9 | 120 | ** |
| $N(COCH_3)CH_3$ | 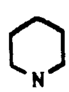 | α | >10 | 10,5 | * | ** |

| | | | | | | |
|---|---|---|---|---|---|---|
| $NH_2$ | 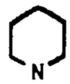 | α | 5,4 | APD shortened | | |
| 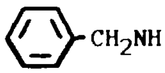 | 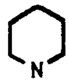 | ß | 1,1 | 3,1 | 66 | 0,9 |
| 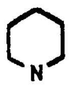 | 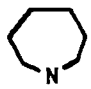 | ß | 5,4 | 1,7 | 16,0 | ** |
| 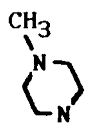 | 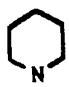 | α | 16,0 | not tested | | |
| $NHC(CH_3)_3$ | $NHC(CH_3)_3$ | α | 2,5 | 19,4 | * | ** |
| 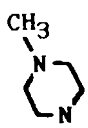 | 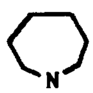 | α | 11,4 | not tested | | |
| $NC(CH_3)_3$<br>$CH_3$ | $NC(CH_3)_3$<br>$CH_3$ | α | 2,1 | not tested | | |

* Maximum prolongation of APD is less than 100%

** Selective class III action

Example 3

| Compositions of tablets: | |
|---|---|
| Compound | Amount (mg/tablet) |
| (2β,3α,5α,16β,17β)-2-amino-16-(1-piperidinyl)-androstane-3,17-diol hydrochloride (1:2) | 250 |
| hydroxypropyl cellulose | 21.0 |
| corn starch | 70.0 |
| magnesium stearate | 5.25 |
| colloidal silicon dioxide | 10.5 |
| lactose 200M | to 700 |

Example 4

| Compositions of tablets: | |
|---|---|
| Compound | Amount (mg/tablet) |
| (2β,3α,5α,16β,17β)-2-amino-16-(1-piperidinyl)-androstane-3,17-diol hydrochloride (1:2) | 150 |
| hydroxypropyl cellulose | 15.75 |
| corn starch | 52.5 |
| magnesium stearate | 3.94 |
| colloidal silicon dioxide | 7.88 |
| lactose 200M | to 525 |

Example 5

| Compositions of tablets: | |
|---|---|
| Compound | Amount (mg/tablet) |
| (2β,3α,5α,16β,17β)-2-(1,1-dimethylethylamino)-16-(1-piperidinyl)-androstane-3,17-diol hydrochloride (1:2) | 150 |
| hydroxypropyl cellulose | 15.75 |
| corn starch | 52.5 |
| magnesium stearate | 3.94 |
| colloidal silicon dioxide | 7.88 |
| lactose 200M | to 525 |

Example 6

| Compositions of tablets: | |
|---|---|
| Compound | Amount (mg/tablet) |
| (2β,3α,5α,16β,17α)-2,16-di-(1,1-dimethyl-ethylamino)-androstane-3,17-diol hydrochloride (1:2) | 150 |
| hydroxypropyl cellulose | 12.6 |
| corn starch | 42 |
| magnesium stearate | 3.15 |
| colloidal silicon dioxide | 6.3 |
| lactose 200M | to 425 |

Example 7

| Composition for an injection preparation | |
|---|---|
| (2β,3α,5α,16β,17β)-2-amino-16-(1-piperidinyl)-androstane-3,17-diol hydrochloride (1:2) | 100 mg |
| mannitol | to isotonicity |
| water for injection | qsad 5 ml |

The composition is placed into a 6 ml vial and freeze-dried.

Example 8

| Composition for an injection preparation | |
|---|---|
| (2β,3α,5α,16β,17β)-2-amino-16-(1-piperidinyl)-androstane-3,17-diol hydrochloride (1:2)<br>$NaH_2PO_4$<br>NaOH<br>mannitol<br>water for injection | 100 mg<br>15 mg<br>to pH 7<br>to isotonicity<br>qsad 5 ml |

The composition is placed into a 6 ml vial and freeze-dried.

Example 9

| Composition for an injection preparation | |
|---|---|
| (2β,3α,5α,16β,17β)-2-amino-16-(1-piperidinyl)-androstane-3,17-diol hydrochloride (1:2)<br>NaCl<br>water for injection | 100 mg<br>to isotonicity<br>qsad 5 ml |

The composition is placed into a 6 ml vial.

**Claims**

**1.** A use of a 2β,16β-diamino-3α,17-dihydroxy androstane derivative of the formula

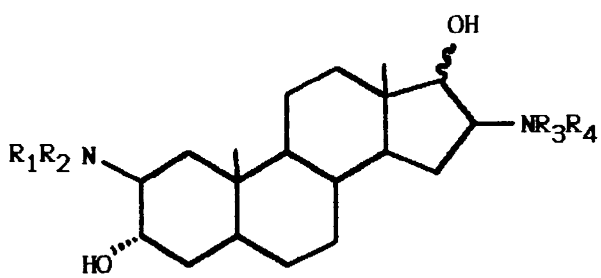

I

in which $R_1$, $R_2$, $R_3$, and $R_4$ are independently selected from hydrogen, $(C_{1-6})$ alkyl, $(C_{7-12})$ aralkyl, $(C_{2-6})$ acyl, and $(C_{3-7})$ cycloalkyl which is optionally substituted with $(C_{1-6})$ alkyl, or $R_1$ and $R_2$, and/or $R_3$ and $R_4$ together with the nitrogen atom to which they are bonded represent a 5-, 6-, or 7-membered ring which is optionally substituted with $(C_{1-6})$ alkyl, and the twitched line represents an α or β bond, or pharmaceutically acceptable salts thereof, for the manufacture of a medicament for treating arrhythmic disorders.

**2.** The use according to claim 1, of the 2β,16β-diamino-3α,17-dihydroxy androstane derivative of formula I in which $R_1$ and $R_2$ are independently selected from hydrogen, $(C_{1-6})$ alkyl, $(C_{7-12})$ aralkyl, $(C_{2-6})$ acyl, and $(C_{3-7})$ cycloalkyl, or $R_1$ and $R_2$ together with the nitrogen atom to which they are bonded represent a pyrrolidinyl, piperidinyl, perhydroazepinyl, or N-methylpiperazinyl, and $R_3$ and $R_4$ are independently selected from hydrogen and $(C_{1-6})$ alkyl, or $R_3$ and $R_4$ together with the nitrogen atom to which they are bonded represent a pyrrolidinyl, piperidinyl, or perhydroazepinyl, and the twitched line represents an α or β bond, or pharmaceutically acceptable salts thereof.

**3.** A 2β,16β-diamino-3α,17-dihydroxy androstane derivative of the formula I, in which $R_1$ and $R_2$ are independently selected from hydrogen, $(C_{1-6})$ alkyl, $(C_{7-12})$ aralkyl, $(C_{2-6})$ acyl, and $(C_{3-7})$ cycloalkyl which is optionally substituted with $(C_{1-6})$ alkyl, and $R_3$ and $R_4$ together with the nitrogen atom to which they are bonded represent a 5-, 6-, or 7-membered ring which is optionally substituted with $(C_{1-6})$ alkyl, and the twitched line represents an α or β bond, or pharmaceutically acceptable salts thereof, for use in therapy.

4. The 2$\beta$,16$\beta$-diamino-3$\alpha$,17-dihydroxy androstane derivative of the formula I, in which $R_1$ is hydrogen, $R_2$ is hydrogen or tert-butyl, $R_3$ and $R_4$ together with the nitrogen atom to which they are bonded represent piperidinyl, and the twitched line is a $\beta$ bond, for use in therapy.

5. The 2$\beta$,16$\beta$-diamino-3$\alpha$,17-dihydroxy androstane derivative according to claim 3 or 4, for the treatment of arrhythmic disorders.

6. A pharmaceutical composition comprising pharmaceutically acceptable auxiliaries and the 2$\beta$,16$\beta$-diamino-3$\alpha$,17-dihydroxy androstane derivative of the formula I, in which $R_1$ and $R_2$ are independently selected from hydrogen, $(C_{1-6})$ alkyl, $(C_{7-12})$ aralkyl, $(C_{2-6})$ acyl, and $(C_{3-7})$ cycloalkyl which is optionally substituted with $(C_{1-6})$ alkyl, and $R_3$ and $R_4$ together with the nitrogen atom to which they are bonded represent a 5-, 6-, or 7-membered ring which is optionally substituted with $(C_{1-6})$ alkyl, and the twitched line represents an $\alpha$ or $\beta$ bond, or pharmaceutically acceptable salts thereof.

7. The pharmaceutical composition comprising pharmaceutically acceptable auxiliaries and the 2$\beta$,16$\beta$-diamino-3$\alpha$,17-dihydroxy androstane derivative of the formula I in which $R_1$ is hydrogen, $R_2$ is hydrogen or *tert*-butyl, $R_3$ and $R_4$ together with the nitrogen atom to which they are bonded represent piperidinyl, and the twitched line is a $\beta$ bond.

European Patent Office

# EUROPEAN SEARCH REPORT

Application Number

EP 91 20 3327

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 113, no. 9, 27th August 1990, page 824, abstrqct no. 78806p, Columbus, Ohio, US; X. XU et al.: "Synthesis of 3alpha-amino-2beta-dihydroxy-16beta-piperidino-5alpha-androstane and 2beta-amino-3alpha,17beta-dihydroxy-16beta-piperidino-5alpha-androstane", & JOUJI HUAXUE 1989, 9(5), 451-4 * Whole document * --- | 3,4,6,7 | A 61 K 31/565<br>A 61 K 31/58 |
| Y | IDEM --- | 1,2,5 | |
| Y | CHEMICAL ABSTRACTS, vol. 74, no. 23, 7th June 1971, page 75, abstract no. 120779k, Columbus, Ohio, US; P.L. SHARMA: "Effect of pancuronium bromide, a new steroidal neuromuscular blocking agent on ventricular arrhythmias in dogs", & INDIAN J. MED. RES. 1970, 58(12), 1736-41 * Whole document * --- | 1,2,5 | **TECHNICAL FIELDS SEARCHED (Int. Cl.5)** |
| D,Y | DE-A-1 593 142 (ORGANON) * Pages 1,2,5; claims * | 1,2,5 | A 61 K |
| D,X | --- | 3,6 | |
| D,Y | EP-A-0 288 102 (AKZO) * Page 2, line 1 - page 3, line 20; claims * | 1,2,5 | |
| D,X | --- -/- | 3,6 | |

The present search report has been drawn up for all claims

| Place of search | Date of completion of the search | Examiner |
|---|---|---|
| THE HAGUE | 04-03-1992 | GOETZ G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application Number

EP 91 20 3327

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | S. BUDAVARI et al.: "The Merck Index. An Encyclopedia of Chemicals, Drugs, and Biologicals", 11th adition, 1989, page 1109, Merck & Co., Inc., Rahway, NJ, US; no. 6958: "Pancuronium bromide" * Whole document * ----- | 1,2,5 | |

TECHNICAL FIELDS SEARCHED (Int. Cl.5)

The present search report has been drawn up for all claims

| Place of search | Date of completion of the search | Examiner |
|---|---|---|
| THE HAGUE | 04-03-1992 | GOETZ G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)